# EUROPEAN PATENT SPECIFICATION

(11) **EP 3 684 458 B1**
(45) Date of publication and mention of the grant of the patent: **12.07.2023**
(21) Application number: 18765132.8
(22) Date of filing: 11.09.2018
(51) Int. Cl.: A61N 1/05, A61N 1/36

(54) **RETINA IMPLANT**
RETINA-IMPLANTAT
IMPLANT RÉTINIEN

(30) Priority: 22.09.2017 LU 100467
(43) Date of publication of application: 29.07.2020
(73) Proprietor: Technische Universität Hamburg-Harburg, 21073 Hamburg (DE)
(72) Inventor: LUJAN VILLARREAL, Diego, 64920 Monterrey, Nuevo Leon (MX); KRAUTSCHNEIDER, Wolfgang, 21149 Hamburg (DE); SCHRÖDER, Dietmar, 29525 Uelzen (DE)
(74) Representative: Hauck Patentanwaltspartnerschaft mbB
(86) International application number: PCT/EP2018/074450
(87) International publication number: WO 2019/057551

(56) References cited:
- EP-A2- 1 762 269
- US-A1- 2008 234 791
- US-A1- 2012 035 726

## Description

The invention relates to a retina implant having an electrode carrier to which a plurality of electrodes are connected, and an electronic controller connected to the electrode carrier for providing stimulation signals to the electrodes.

Retina implants of this type, also referred to as visual prostheses, include a plurality of electrodes which are implanted within an eye of a patient, so that electrical stimulation signals applied to the electrodes activate nearby retinal nervous cells, in particular ganglion cells. Electrical stimulation of retinal ganglion cells was shown to create visual sensations named phosphenes. Apparently, this approach is suitable for patients suffering from various diseases affecting in particular photoreceptor mechanisms of the retina, while leaving the ganglionic cells intact, such as retinitis pigmentosa or other inherited retina degenerations.

The electronic controller of a retina implant is combined with a camera or other technical means for converting incoming light into electrical signals. These technical means may include a CCD or other photodetectors. External cameras may be used, as well as photodetectors attached to or integrated within an electrode carrier implanted into the eye.

The document US 2004/0082981 A1 shows an example of a micro photodetector retina implant having a two-dimensional carrier surface carrying a plurality of electrodes arranged in a rectangular grid. The photodetectors are integrated with the carrier surface, surrounding the electrodes.

The document US 2008/0228242 A1 describes another retina implant having an electrode array implanted within an eye and connected to an electronic controller. The electronic controller is connected to an external camera including an image processor via a wireless interface.

The document EP 1 762 269 A2 discloses a retinal implant whose electrodes are designed so that they conform to the retinal curvature.

The document US 2012/0035726 A1 discloses a retina implant with the features of the preamble of claim 1.

The scientific publication "Interim results of a multicenter trial with the new electronic subretinal implant Alpha AMS in 15 patients blind from inherited retinal degenerations" by Katarina Stingl et al., Frontiers in Neuroscience, August 2017, volume 11, article 445, describes a retina implant having an electrode carrier comprising a CMOS chip attached to a polyimide foil which is implanted sub-retinally. The chip encompasses 1,600 pixel cells. Each pixel cell includes a photodiode, an amplifier and a stimulation electrode.

While it has been shown that at least limited visual functions in blind patients with degenerations of the outer retina can be restored with the aforementioned technologies, there remains room for improvement, among other things, with regard to the achievable resolution. Based on this, it is the technical problem of the invention to provide a retina implant allowing for improved resolution of the visual perceptions.

This problem is solved by the retina implant with the features of claim 1. Advantageous aspects of the invention are given in the dependent claims.

The retina implant comprises
- an electrode carrier having a two-dimensional carrier surface and being adapted for surgical implantation within an eye such that the carrier surface faces a layer of a retina,
- a plurality of electrodes attached to the carrier surface, and
- an electronic controller connected to the electrodes for providing stimulation signals to the electrodes, wherein
- the electrodes are arranged in varying distances from the carrier surface, and wherein
- the carrier surface (12) comprises a plurality of electrode connecting points (14) arranged in a pattern on the carrier surface (12), each of the electrode connecting points (14) being connected to at least one of the electrodes (18), wherein
- a plurality of linear carrier elements (16), each of the linear carrier elements (16) being connected to an electrode connecting point (14) and carrying a plurality of the electrodes (18).

The electrode carrier may have a generally plate-like shape. It may comprise a rigid and/or stiff material, such as a semiconducor substrate, or a flexible material such as a polymeric film. The thickness of the electrode carrier may be small compared to its lateral dimensions. The overall shape of the electrode carrier may be circular or rectangular, for example. The two-dimensional carrier surface may be flat or curved in one or two dimensions.

In use, the electrode carrier including the plurality of electrodes attached to the carrier surface is implanted within a patient's eye such that the carrier surface faces a layer of a retina. This layer in particular is a ganglionic layer of the retina. However, it may be an option to implant the electrode carrier such that the carrier surface faces another layer of the retina, either a layer including nervous cells or any other layer of the retina.

The electronic controller is connected to the electrodes for providing stimulation signals to the electrodes. The electronic controller may be attached to or formed integral with the electrode carrier. In this case, the electronic controller will be implanted at the retina. As an alternative, the electronic controller may be connected to the electrode carrier via one or more cables. In this case, the electronic controller may still be implanted within a patient's eye, for example at the retina and in a certain distance from the electrode carrier, or at any other location within or external to the eye.

The electrode controller can be adapted to control the characteristics of the stimulation signals, in particular a shape of a stimulating pulse, an amplitude of the electrical current of a stimulating pulse, and/or a selection of a first set of electrodes functioning as active electrodes and/or a selection of a second set of electrodes functioning as ground electrodes. This gives flexibility in defining effective and safe stimulation signals under different conditions.

Independent of its position, the electronic controller is adapted to provide stimulation signals to the electrodes. In particular, the electronic controller is adapted to address each one of the electrodes, one or more at a time, as well as in a consecutive manner. It is understood that the stimulation signals will be controlled by the electronic controller in order to create phosphenes, in particular in accordance with a pattern of light incident onto a receiver interfaced with the electronic controller. As mentioned earlier, the light receiver may include for example a camera with a CCD or any other type of photodetectors. The light receiver may form a part of the retina implant implanted within the eye as well or it may be placed externally and connected to the electronic controller wirelessly, for example.

In accordance with the invention, the electrodes are arranged in varying distances from the carrier surface. This means that the electrodes are positioned at least in part outside of the electrode carrier so that when implanted, they will be positioned within the layer of the retina at which the carrier surface is implanted. Arranged in varying distances from the carrier surface means the plurality of electrodes are distributed over a certain volume which may extend from the carrier surface into the retinal layer.

The inventors observed that conventional retina implants with electrodes arranged in a two-dimensional pattern tend to create phosphenes of varying shapes, including relatively small circular shapes, larger circular shapes, hashmark-shapes, rectangular shapes and even ring shapes. Extensive computer simulations analyzing activation areas within the retina based on current densities suggested that the shape of phosphenes is, among other things, influenced by the distance of the electrodes from the relevant nervous cells. Even with the smallest electrodes, it may not always be possible to target a certain ganglionic cell without activating many other, adjacent ganglionic cells as well.

With the inventive, three-dimensional arrangement of the electrodes it is possible to narrow the activation area so that a smaller number of ganglionic cells or even a single ganglionic cell can be activated selectively. Without wishing to be bound by theory, it is believed that this in part is due to the fact that potential differences and corresponding current paths between different electrodes can be limited to smaller volumes within the retinal tissue. In particular, one may find suitable combinations of electrodes being positioned at different sides of a certain ganglionic cell, so that the delivering electrical current between these electrodes runs more or less directly through the respective ganglionic cell. In contrast to this theoretically ideal situation, conventional retina implants having electrode configurations with active and ground electrodes arranged more or less in the same plane will always lead to curved current paths reaching out from a first electrode at the carrier surface into the retinal tissue and back again to the other electrode at the carrier surface.

Another advantage of the three-dimensional arrangement of the electrodes is that by selecting suitable electrodes one may compensate for positioning tolerances occurring when implanting the electrode carrier.

According to an aspect of the invention, at least one of the electrodes is positioned in a minimum distance from the carrier surface, the minimum distance in particular being less than 10 µm. In particular, the minimum distance from the carrier surface may be zero or almost zero when the electrodes closest to the carrier surface are placed directly at the carrier surface. However, it may also be desired to use a minimum distance larger than zero, so that for placing the closest electrodes in the proximity of ganglionic cells, the carrier surface can be placed in a certain distance from the ganglionic layer. This distance may help to accommodate for additional tissue layers and/or for inevitable spacings.

According to an aspect of the invention, at least one of the electrodes is positioned in a maximum distance from the carrier surface, the maximum distance being more than 50 µm. This allows to cover a substantial part of the respective retinal layer by the volume arranged between the closest and most distal electrodes. In particular, the electrodes placed in the maximum distance from the carrier surface will penetrate sufficiently deeply into the retinal layer if the electrode carrier is implanted in the intended manner.

In accordance with the invention, the carrier surface comprises a plurality of electrode connecting points arranged in a pattern on the carrier surface, each of the electrode connecting points being connected to at least one of the electrodes. In particular, each of the electrode connecting points will be connected to more than one of the electrodes, for example to a number of electrodes ranging from 3 to 50. To allow for selective application of each of these several electrodes connected to one electrode connecting point, the electrode connecting point may include a corresponding number of contacts. The pattern in which the electrode connecting points are arranged on the carrier surface may be a regular pattern, in particular a rectangular grid of electrode connecting points.

In accordance with the invention, there is provided a plurality of linear carrier elements, each of the linear carrier elements being connected to an electrode connecting point and carrying a plurality of the electrodes. The electrodes carried by a linear carrier element may be arranged along a substantially straight line, or along a curved trajectory. In any event, the linear carrier elements can facilitate the desired placement of the electrodes within the respective retinal layer. The linear carrier elements may penetrate into or through this layer. The linear carrier element may be made from a flexible material such as a polymeric film. It may be made from a semiconducting material, in particular silicon. The linear carrier element may be made of an electrically conducting or electrically isolating material. The dimensions and mechanical properties of the linear carrier element may be selected such that it can be placed within the retinal tissue without causing substantial damage of the nervous cells. The linear carrier element may include one or more conductive paths, such as electrically conductive filaments or other electrical conductors. The conductive paths may be electrically isolated from each other and/or from the surrounding environment. Each of the conductive paths may be electrically connected to a single one of the electrodes.

According to an aspect of the invention, the linear carrier elements each comprise a wire, wherein the plurality of the electrodes carried by the respective linear carrier element are affixed to the wire. The wire serves to provide sufficient mechanical stability to the linear carrier element, so that the same can be placed within the respective retinal tissue in a desired position.

According to an aspect of the invention, each linear carrier element comprises a plurality of decoding circuits, each of the decoding circuits being assigned to one of the electrodes of the corresponding linear carrier element, and a fixed number of conductive paths which are all connected to each of the decoding circuits. Each of the decoding circuits may be arranged close to an electrode. Each electrode of a linear carrier element can be addressed via the same fixed number of conductive paths. In particular, each of the electrodes may comprise a decoding circuit adapted to connect the corresponding electrode to a current source or to ground under the control of a logic signal sent via one or more of the conductive paths from the electronic controller and decoded in the decoding circuit. For example, there may be four conductive paths on each linear carrier element, one of them connected to a current source, one of them connected to ground, one of them for transferring a control signal to address a certain electrode and one of them for the power supply for the decoding circuit. For a more robust noise signal suppression, additional conductive paths can be used for e.g. differential scheme implementation and for enabling the decoding circuit.

According to an aspect of the invention, the electrodes of at least one of the linear carrier elements are distributed over an electrode segment of the respective linear carrier element in regular intervals. For example, the electrode segment may extend over the entire length of the linear carrier element so that the electrodes cover the entire length of the linear carrier element extending from the carrier surface. In the alternative, the electrode segment may form only a part of the linear carrier element so that the electrodes may cover a certain range of distances from the carrier surface only. In this case, the linear carrier element may include a holding segment extending from the carrier surface to the electrode segment.

According to an aspect of the invention, at least one of the electrode segments is arranged in a direction normal to the carrier surface. This means the electrodes of the electrode segment are arranged in varying distances from the carrier surface.

According to an aspect of the invention, at least one of the electrode segments is arranged in a constant distance from the carrier surface. This means each of the electrodes of this electrode segment are placed at the same distance from the carrier surface, while other electrodes possibly arranged in other electrode segments are placed in smaller or larger distances from the carrier surface.

According to an aspect of the invention, the carrier surface forms a portion of a sphere. In general, the two-dimensional carrier surface may as well be flat or almost flat or it may be curved in one or two dimensions. When the carrier surface forms a portion of a sphere, reflecting the shape of a corresponding portion of the retina, this may facilitate placing the carrier surface closely to the retinal layer.

According to an aspect of the invention, the electrode carrier comprises a flexible foil. This can facilitate placing the carrier surface closely to the respective layer of the retina as well.

In an aspect of the invention, the electronic controller is adapted to provide a stimulation signal by applying a current pulse to at least one first electrode of the plurality of electrodes and by connecting at least one second electrode of the plurality of electrodes to ground. In other words, the plurality of electrodes covering a certain volume of retinal tissue includes electrodes serving as active electrodes as well as electrodes serving as ground electrodes. The electronic controller is connected to the plurality of electrodes in such a manner that selected electrodes can be used for this purpose.

According to an aspect of the invention, the electronic controller is adapted to use each of the electrodes as a first electrode and as a second electrode. This offers even greater flexibility when selecting certain combinations of electrodes for establishing a current path.

According to an aspect of the invention, the first electrode is connected to a first electrode connecting point of the plurality of electrode connecting points and the second electrode is connected to a second electrode connecting point of the plurality of electrode connecting points. In this situation a current path is formed between different electrode connecting points, that is for example between different linear carrier elements or electrode segments.

According to an aspect of the invention, the first and second electrodes are connected to the same electrode connecting point. In this situation, a current path is provided between electrodes for example arranged on the same linear carrier element or electrode segment.

In the following, the invention is explained in greater detail based on several embodiments shown in figures. The figures show:
- Fig. 1: a first embodiment of a retina implant in a schematic view,
- Fig. 2: the retina implant of Fig. 1 implanted into a human eye in schematic view,
- Fig. 3: a perspective view on the electrode carrier of the embodiment shown in Fig. 2,
- Fig. 4: an electrode carrier of another embodiment in a perspective view corresponding to Fig. 3,
- Fig. 5a: a top perspective view of a linear carrier element carrying a plurality of circular electrodes,
- Fig. 5b: the linear carrier element of Fig. 5a in a bottom perspective view,
- Fig. 5c: the linear carrier element of Fig. 5a in a side face perspective view,
- Fig. 5d: top perspective view of a linear carrier element carrying a plurality of rectangular electrodes,
- Fig. 5e: the linear carrier element of Fig. 5d in a bottom perspective view,
- Fig. 5f: the linear carrier element of Fig. 5d in a side face perspective view,
- Fig. 6a: the linear carrier element of Fig. 5a in a schematic view showing the decoding circuits connected with the conductive paths and the corresponding electrode.
- Fig. 6b: the linear carrier element of Fig. 5d in a schematic view showing the decoding circuit elements connected with the conductive paths and the corresponding electrode.

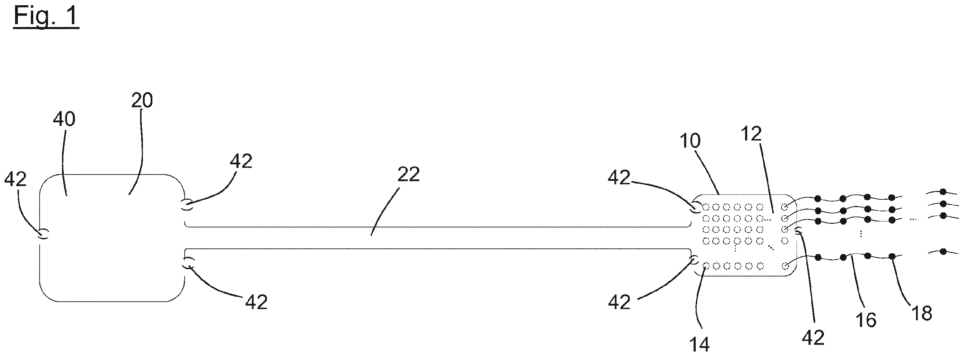

The retina implant of figure 1 includes an electrode carrier 10 having a substantially rectangular, plate-like shape. A two-dimensional carrier surface 12 is directed towards the viewer and comprises a plurality of electrode connecting points 14 arranged in a rectangular matrix.

As shown in figure 1 for some of the right-most electrode connecting points 14 only, each electrode connecting point 14 is connected to a linear carrier element 16 formed by a wire. Each of the linear carrier elements 16 carries a plurality of electrodes 18 placed in substantially regular intervals.

The electrodes carrier 10 is connected to an electronic controller 20 via a microcable 22. This connection is carried out such that the electronic controller 20 can provide stimulation signals selectively to each of the electrodes 18.

Although not shown in detail in figure 1, the electronic controller 20, which may be referred to as a stimulation chip as well, is combined with receiver electronics 40. Via the receiver electronics 40, wireless communication with an external camera can be accomplished.

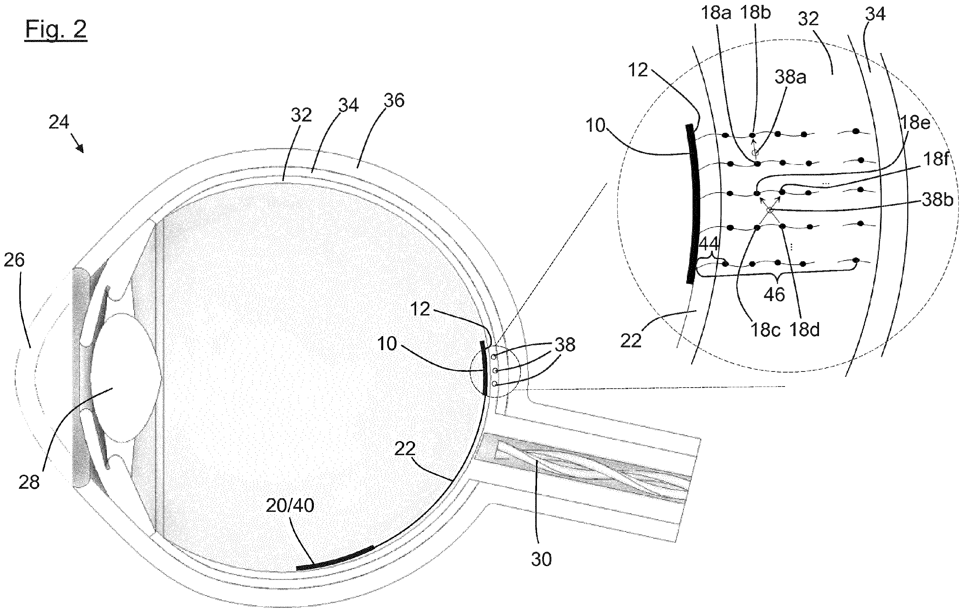

Figure 2 is a simplified cross-section showing the retina implant of figure 1 implanted into a human eye 24. The eye 24 includes a cornea 26, a lens 28 and an optic nerve 30. The retina 32 forms a part of a sphere surrounded by the choroid 34 and the sclera 36. The retina 32 includes several layers including a ganglionic layer in which the ganglionic cells 38 are disposed.

Approximately within the optical axis and at the fovea not shown in the figure, the electrode carrier 10 is implanted such that its carrier surface 12 faces a layer of the retina 32 which includes the ganglionic cells 38. Away from the optical axis and in a distance from the electrode carrier 10, the electronic controller 20 and the receiver electronics 40 are implanted within the eye 24. Both the electrode carrier 10 and the electronic controller 20 with the receiver electronics 40 are attached to the retina by means of a suitable number of tacks 42 illustrated schematically in figure 1.

The microcable 22 connecting the electrode carrier 10 with the electronic controller 20 is placed within the eye 24, close to the retina 32.

As best seen in the enlarged view to the top right of Fig. 1, the linear carrier elements 16 extend from the carrier surface 12 of the electrode carrier 10 in a direction normal to the carrier surface 12 into the retina 32, in particular into the ganglionic layer thereof. It can be seen that the electrodes 18 form a three-dimensional array distributed over a certain volume of the respective layer of the retina 32.

For stimulating ganglionic cells 38 of the retina 32, the electronic controller 20 may follow different strategies. According to a single-site strategy, a ganglionic cell 38a may be stimulated by using electrode 18a of one of the linear carrier elements 16 as a first, active electrode, and electrode 18b of another linear carrier element 16 as a ground electrode. According to a multi-site strategy, a ganglionic cell 38b may be stimulated by using two or more first electrodes, for example 18c, 18d (possibly disposed on a certain linear carrier element 16 as shown, or disposed on different linear carrier elements 16) as first/active electrodes and two or more adjacent, second carrier electrodes for example 18e, 18f (possibly disposed on a certain linear carrier element 16 as shown, or disposed on different linear carrier elements 16) as ground electrodes. Current pulses indicated by small arrows may in each case lead to an activation of the targeted ganglionic cell 38a, 38b. In addition, this scheme gives the flexibility to select a desired stimulating pulse, for example charge-balanced biphasic rectangular pulse, and the require amplitude of electrical current for effective stimulation of retinal neurons.

As indicated in Fig. 2 by the curly braces, some of the electrodes 18 are positioned in a minimum distance 44 from the carrier surface 12, while other electrodes are positioned in a maximum distance 46 from the carrier surface 12.

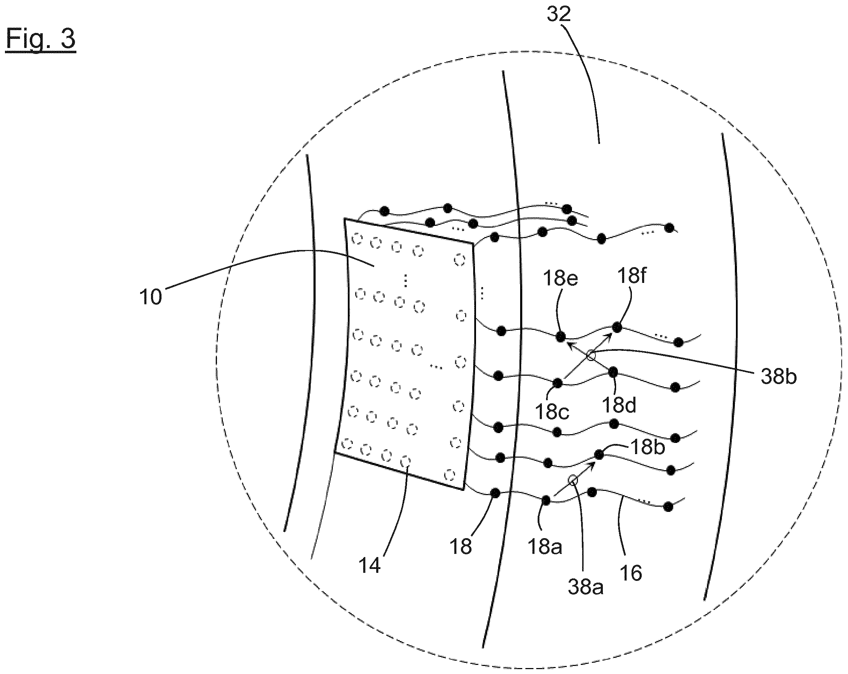

Figure 3 shows the elements of the enlarged section of Fig. 2 in a perspective view. The elements explained with regard to Fig. 2 are provided with the same reference numerals. As can be seen more clearly in Fig. 3, the electronic carrier 10 and its carrier surface 12 have a generally rectangular shape, wherein the carrier surface 12 forms a part of a sphere to correspond to the shape of the retina 32. One can also see from Fig. 3 that the various electrodes 18 are distributed over a three-dimensional volume.

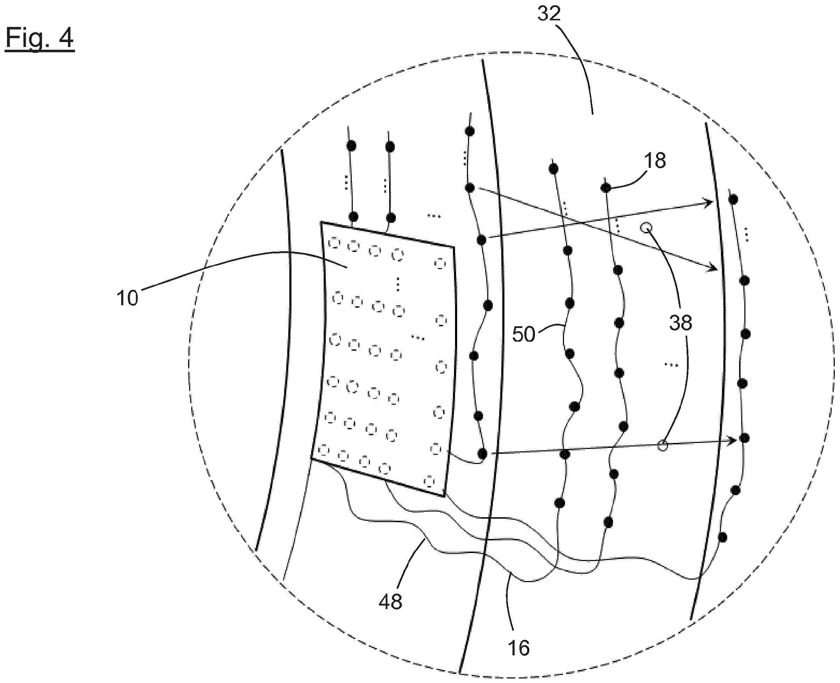

Figure 4 shows another electrode carrier 10 in a view similar to the view of Fig. 3, that is in a perspective view when implanted into a human eye 24. In this embodiment, each of the linear carrier elements 16 includes a holding segment 48 and an electrode segment 50, wherein only the electrode segment 50 carries the electrodes 18 which are again arranged in substantially regular intervals. In contrast to the orientation normal to the carrier surface 12 shown in the first embodiment, the electrode segments 50 of Fig. 4 are oriented parallel to the carrier surface 12, so that the various electrodes 18 carried by one of the electrode segments 50 are all placed within approximately the same distance from the carrier surface 12. However, similar to the embodiment of Fig. 3, the embodiment of Fig. 4 exhibits a certain three-dimensional volume over which the electrodes 18 are distributed, again covering varying distances from the carrier surface 12. This gives the advantage of including different current paths along the ganglionic layer for the sake of activating single cells and improving visual perception.

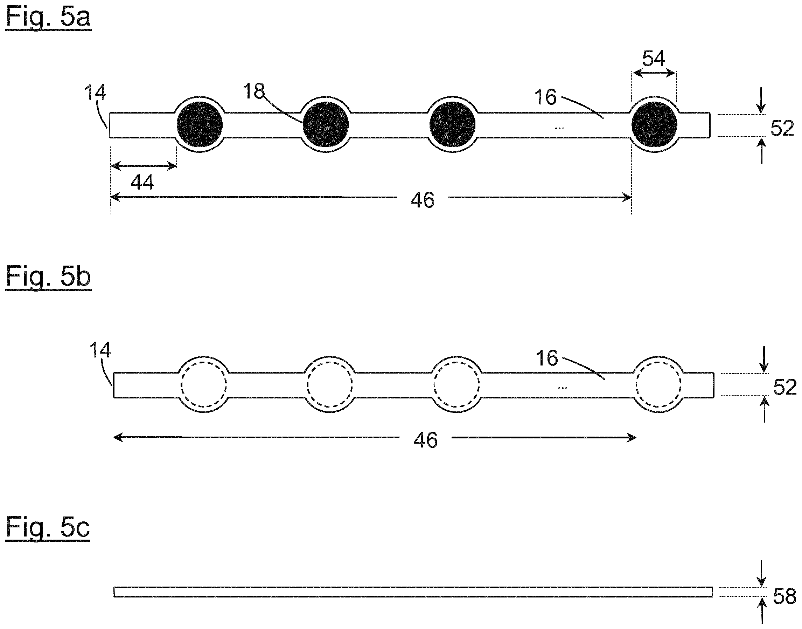

Figs. 5a, 5b and 5c show views of top, bottom and side face of a linear carrier element 16 which carries a plurality of electrodes 18 intended to deliver current pulses to the underlying retinal ganglion cells for activation. To the left of these figures, the linear carrier elements 16 are connected to an electrode connecting point 14 of an electrode carrier 10, for example the electrode carrier 10 shown in figure 1. The leftmost electrode 18 is disposed in minimum distance 44 from the carrier surface 12 or the electrode connecting point 14, respectively. The rightmost electrode 18 is disposed in a maximum distance 46 from the electrode carrier 10 or the carrier surface 12, respectively.

Linear carrier element 16 may comprise a flexible material such as polymeric film with a small thickness 58 and/or a small width 52 compared to its length, in order to comply with bending requirements inside the ganglionic layer.

State-of-the-art technology can realize a narrow width 52 of the linear carrier element 16 of around 1 µm with the proper conductive paths 62 and electrode connections 56. Extensive computer simulations analyzing activation areas at the surface of the retina suggested that an electrode diameter twice of the width, i.e. 2 µm, can deliver safe stimulus within a distance of 10 µm of the electrodes 18 from the relevant nervous cells.

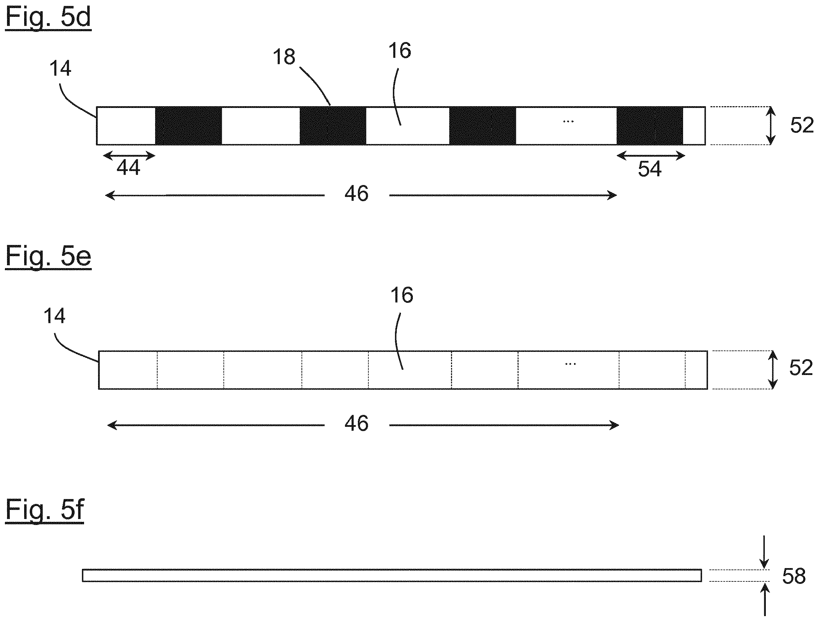

Figs. 5d, 5e and 5f show another linear carrier element 16 with similar features as discussed with regard to Figs. 5a, 5b and 5c. However, the electrodes 18 differ in their geometric shape which is not circular, but rectangular. Also, the rectangular electrodes 18 have a width corresponding to the width 52 of the linear carrier element 16.

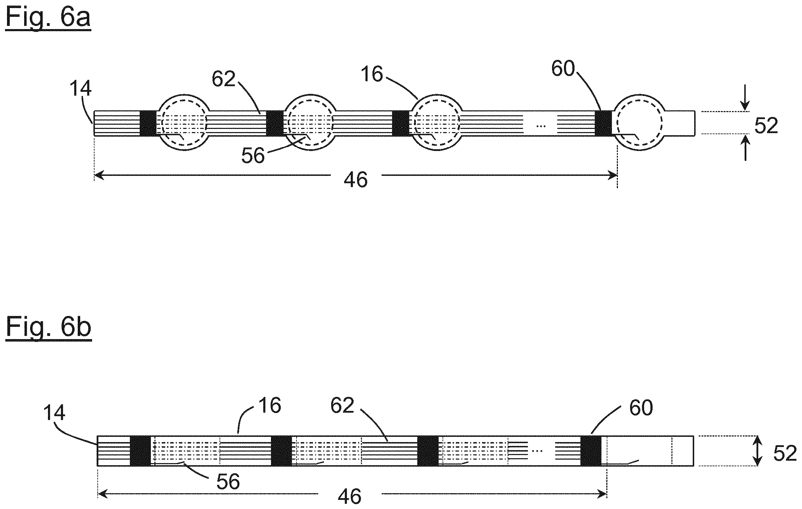

Fig. 6a show the bottom perspective view of the linear carrier element 16 of Fig. 5a with a fixed number of conductive paths 62 connected to each of the decoding circuits 60 that select the corresponding electrode 18 which is connected via the electrode connection 56. This figure shows that each of the electrodes 18 is connected to the electrode connecting point 14 by means of a separate electrode connection 56 from a decoding circuit 60. In the example, the electrode connections 56 and conductive paths 62 are formed from small metallic conductive paths arranged at the bottom of the linear carrier element 16.

Fig. 6b show another linear carrier element 16 with similar features as discussed with regard to Fig. 6a. However, the electrodes 18 differ in their geometrical shape which is not circular, but rectangular.

### List of reference numerals:

- 10: electrode carrier
- 12: carrier surface
- 14: electrode connecting point
- 16: linear carrier element
- 18: electrode
- 20: electronic controller (stimulation chip)
- 22: microcable
- 24: eye
- 26: cornea
- 28: lens
- 30: optic nerve
- 32: retina
- 34: choroid
- 36: sclera
- 38: ganglionic cell
- 40: receiver electronics
- 42: tack
- 44: minimum distance
- 46: maximum distance
- 48: holding segment
- 50: electrode segment
- 52: width of linear carrier element
- 54: electrode diameter/length
- 56: electrode connection
- 58: thickness of linear carrier element
- 60: decoding circuit for addressing electrodes
- 62: conductive paths

## Claims

1. A retina implant comprising
• an electrode carrier (10) having a two-dimensional carrier surface (12) and being adapted for surgical implantation within an eye such that the carrier surface (12) faces a layer of a retina (32),
• a plurality of electrodes (18) attached to the carrier surface (12), and
• an electronic controller (20) connected to the electrodes (18) for providing stimulation signals to the electrodes (18), wherein
• the electrodes (18) are arranged in varying distances from the carrier surface (12), and wherein
• the carrier surface (12) comprises a plurality of electrode connecting points (14) arranged in a pattern on the carrier surface (12), each of the electrode connecting points (14) being connected to at least one of the electrodes (18), **characterized by**
• a plurality of linear carrier elements (16), each of the linear carrier elements (16) being connected to an electrode connecting point (14) and carrying a plurality of the electrodes (18).

2. A retina implant according to claim 1, **characterized in that** at least one of the electrodes (18) is positioned at a minimum distance (44) of less than 10 µm from the carrier surface (12).

3. A retina implant according to claim 1 or 2, **characterized in that** at least one of the electrodes (18) is positioned at a maximum distance (46) greater than 50 µm from the carrier surface (12).

4. A retina implant according to any of the claims 1 to 3, **characterized in that** the linear carrier elements (16) each comprise a wire, wherein the plurality of the electrodes (18) carried by the respective linear carrier element (16) are affixed to the wire.

5. A retina implant according to any of the claims 1 to 4, **characterized in that** each linear carrier element (16) comprises a plurality of decoding circuits (60), each of the decoding circuits (60) being assigned to one of the electrodes (18) of the corresponding linear carrier element (16), and a fixed number of conductive paths (62) which are all connected to each of the decoding circuits (60).

6. A retina implant according to any of the claims 1 to 5, **characterized in that** the electrodes (18) of at least one of the linear carrier element (16) are distributed over an electrode segment (50) of the respective linear carrier element (16) in regular intervals.

7. A retina implant according to any of the claims 1 to 6, **characterized in that** at least one of the electrode segments (50) is arranged in a direction normal to the carrier surface (12).

8. A retina implant according to any of the claims 1 to 7, **characterized in that** at least one of the electrode segments (50) is arranged in a constant distance from the carrier surface (12).

9. A retina implant according to any of the claims 1 to 8, **characterized in that** the carrier surface (12) forms a portion of a sphere.

10. A retina implant according to any of the claims 1 to 9, **characterized in that** the electrode carrier (10) comprises a flexible foil.

11. A retina implant according to any of the claims 1 to 10, **characterized in that** the electronic controller (20) is adapted to provide a stimulation signal by applying a current pulse to at least one first electrode (18a, 18c, 18d) of the plurality of electrodes (18) and by connecting at least one second electrode (18b, 18e, 18f) of the plurality of electrodes (18) to ground.

12. A retina implant according to claim 11, **characterized in that** the electronic controller (20) is adapted to use each of the electrodes (18) as a first electrode (18a, 18c, 18d) and as a second electrode (18b, 18e, 18f).

13. A retina implant according to claim 11 or 12, **characterized in that** the first electrode (18a, 18c, 18d) is connected to a first electrode connecting point (14) of the plurality of electrode connecting points (14) and the second electrode (18b, 18e, 18f) is connected to a second electrode connecting point (14) of the plurality of electrode connecting points (14).

14. A retina implant according to claim 11 or 12, **characterized in that** the first and second electrodes are connected to the same electrode connecting point (14).

## Patentansprüche

1. Retina-Implantat, umfassend:
• einen Elektrodenträger (10), der eine zweidimensionale Trägeroberfläche (12) aufweist und zur chirurgischen Implantation innerhalb eines Auges angepasst ist, sodass die Trägeroberfläche (12) einer Schicht einer Retina (32) zugewandt ist,
• eine Vielzahl von Elektroden (18), die an der Trägeroberfläche (12) angebracht sind, und
• eine elektronische Steuereinrichtung (20), die mit den Elektroden (18) verbunden ist, zum Bereitstellen von Stimulationssignalen für die Elektroden (18), wobei
• die Elektroden (18) in veränderlichen Abständen von der Trägeroberfläche (12) angeordnet sind, und wobei
• die Trägeroberfläche (12) eine Vielzahl von Elektrodenverbindungspunkten (14) umfasst, die in einem Muster auf der Trägeroberfläche (12) angeordnet sind, wobei jeder der Elektrodenverbindungspunkte (14) mit mindestens einer der Elektroden (18) verbunden ist, **gekennzeichnet durch**
• eine Vielzahl von linearen Trägerelementen (16), wobei jedes der linearen Trägerelemente (16) mit einem Elektrodenverbindungspunkt (14) verbunden ist und eine Vielzahl von den Elektroden (18) trägt.

2. Retina-Implantat nach Anspruch 1, **dadurch gekennzeichnet, dass** mindestens eine der Elektroden (18) in einem Mindestabstand (44) von weniger als 10 µm von der Trägeroberfläche (12) positioniert ist.

3. Retina-Implantat nach Anspruch 1 oder 2, **dadurch gekennzeichnet, dass** mindestens eine der Elektroden (18) in einem Höchstabstand (46) von größer als 50 µm von der Trägeroberfläche (12) positioniert ist.

4. Retina-Implantat nach einem der Ansprüche 1 bis 3, **dadurch gekennzeichnet, dass** die linearen Trägerelemente (16) jeweils einen Draht umfassen, wobei die Vielzahl von den Elektroden (18), die von dem entsprechenden linearen Trägerelement (16) getragen werden, an dem Draht befestigt sind.

5. Retina-Implantat nach einem der Ansprüche 1 bis 4, **dadurch gekennzeichnet, dass** jedes lineare Trägerelement (16) eine Vielzahl von Decodierungsschaltungen (60), wobei jede der Decodierungsschaltungen (60) einer der Elektroden (18) des entsprechenden linearen Trägerelements (16) zugewiesen ist, und eine feste Anzahl von Leiterbahnen (62) umfasst, die alle mit jeder der Decodierungsschaltungen (60) verbunden sind.

6. Retina-Implantat nach einem der Ansprüche 1 bis 5, **dadurch gekennzeichnet, dass** die Elektroden (18) von mindestens einem von dem linearen Trägerelement (16) in gleichmäßigen Intervallen über ein Elektrodensegment (50) des entsprechenden linearen Trägerelements (16) verteilt sind.

7. Retina-Implantat nach einem der Ansprüche 1 bis 6, **dadurch gekennzeichnet, dass** mindestens eines der Elektrodensegmente (50) in einer Richtung normal zur Trägeroberfläche (12) angeordnet ist.

8. Retina-Implantat nach einem der Ansprüche 1 bis 7, **dadurch gekennzeichnet, dass** mindestens eines der Elektrodensegmente (50) in einem konstanten Abstand von der Trägeroberfläche (12) angeordnet ist.

9. Retina-Implantat nach einem der Ansprüche 1 bis 8, **dadurch gekennzeichnet, dass** die Trägeroberfläche (12) einen Abschnitt einer Kugel bildet.

10. Retina-Implantat nach einem der Ansprüche 1 bis 9, **dadurch gekennzeichnet, dass** der Elektrodenträger (10) eine biegsame Folie umfasst.

11. Retina-Implantat nach einem der Ansprüche 1 bis 10, **dadurch gekennzeichnet, dass** die elektronische Steuereinrichtung (20) so angepasst ist, dass sie ein Stimulationssignal durch Anlegen eines Stromimpulses an mindestens eine erste Elektrode (18a, 18c, 18d) der Vielzahl von Elektroden (18) und durch Verbinden mindestens einer zweiten Elektrode (18b, 18e, 18f) der Vielzahl von Elektroden (18) mit der Masse bereitstellt.

12. Retina-Implantat nach Anspruch 11, **dadurch gekennzeichnet, dass** die elektronische Steuereinrichtung (20) so angepasst ist, dass sie jede der Elektroden (18) als eine erste Elektrode (18a, 18c, 18d) und als eine zweite Elektrode (18b, 18e, 18f) verwendet.

13. Retina-Implantat nach Anspruch 11 oder 12, **dadurch gekennzeichnet, dass** die erste Elektrode (18a, 18c, 18d) mit einem ersten Elektrodenverbindungspunkt (14) der Vielzahl von Elektrodenverbindungspunkten (14) verbunden ist und die zweite Elektrode (18b, 18e, 18f) mit einem zweiten Elektrodenverbindungspunkt (14) der Vielzahl von Elektrodenverbindungspunkten (14) verbunden ist.

14. Retina-Implantat nach Anspruch 11 oder 12, **dadurch gekennzeichnet, dass** die erste und die zweite Elektrode mit demselben Elektrodenverbindungspunkt (14) verbunden sind.

## Revendications

1. Implant rétinien comprenant
• un support d'électrode (10) ayant une surface de support bidimensionnelle (12) et étant conçu pour une implantation chirurgicale dans un oeil de telle sorte que la surface de support (12) soit en face d'une couche de rétine (32),
• une pluralité d'électrodes (18) attachées à la surface de support (12), et
• un dispositif de commande électronique (20) connecté aux électrodes (18) pour fournir aux électrodes (18) des signaux de stimulation,
• les électrodes (18) étant disposées à des distances variables de la surface de support (12), et
• la surface de support (12) comprenant une pluralité de points de connexion d'électrode (14) disposés en un motif sur la surface de support (12), chacun des points de connexion d'électrode (14) étant connecté à l'au moins une des électrodes (18), **caractérisé par**
• une pluralité d'éléments de support linéaires (16), chacun des éléments de support linéaires (16) étant connecté à un point de connexion d'électrode (14) et supportant une pluralité des électrodes (18).

2. Implant rétinien selon la revendication 1, **caractérisé en ce qu'**au moins l'une des électrodes (18) est positionnée à une distance minimum (44) de la surface de support (12) inférieure à 10 µm.

3. Implant rétinien selon la revendication 1 ou 2, **caractérisé en ce qu'**au moins l'une des électrodes (18) est positionnée à une distance maximum (46) de la surface de support (12) supérieure à 50 µm.

4. Implant rétinien selon l'une quelconque des revendications 1 à 3, **caractérisé en ce que** les éléments de support linéaires (16) comprennent chacun un fil, la pluralité des électrodes (18) portées par l'élément de support linéaire (16) respectif étant fixées sur le fil.

5. Implant rétinien selon l'une quelconque des revendications 1 à 4, **caractérisé en ce que** chacun des éléments de support linéaires (16) comprend une pluralité de circuits de décodage (60), chacun des circuits de décodage étant attribué à l'une des électrodes (18) de l'élément de support linéaire (16) correspondant, et un nombre fixe de chemins conducteurs (62) qui sont tous connectés à chacun des circuits de décodage (60).

6. Implant rétinien selon l'une quelconque des revendications 1 à 5, **caractérisé en ce que** les électrodes (18) d'au moins un des éléments de support linéaires (16) sont distribuées sur un segment d'électrode (50) de l'élément de support linéaire (16) respectif à intervalles réguliers.

7. Implant rétinien selon l'une quelconque des revendications 1 à 6, **caractérisé en ce qu'**au moins un des segments d'électrodes (50) est disposé dans une direction perpendiculaire à la surface de support (12).

8. Implant rétinien selon l'une quelconque des revendications 1 à 7, **caractérisé en ce qu'**au moins un des segments d'électrodes (50) est disposé à une distance constante par rapport à la surface de support (12).

9. Implant rétinien selon l'une quelconque des revendications 1 à 8, **caractérisé en ce que** la surface de support (12) forme une partie d'une sphère.

10. Implant rétinien selon l'une quelconque des revendications 1 à 9, **caractérisé en ce que** le support d'électrode (10) comprend une feuille souple.

11. Implant rétinien selon l'une quelconque des revendications 1 à 10, **caractérisé en ce que** le dispositif de commande électronique (20) est conçu pour fournir un signal de stimulation en appliquant une impulsion de courant à au moins une première électrode (18a, 18c, 18d) de la pluralité d'électrodes (18) et en connectant à la terre au moins une deuxième électrode (18b, 18e, 18f) de la pluralité d'électrodes (18).

12. Implant rétinien selon la revendication 11, **caractérisé en ce que** le dispositif de commande électronique (20) est conçu pour utiliser chacune des électrodes (18) comme une première électrode (18a, 18c, 18d) et comme une deuxième électrode (18b, 18e, 18f).

13. Implant rétinien selon la revendication 11 ou 12, **caractérisé en ce que** la première électrode (18a, 18c, 18d) est connectée à un premier point de connexion d'électrode (14) de la pluralité de points de connexion d'électrode (14) et **en ce que** la deuxième électrode (18b, 18e, 18f) est connectée à un deuxième point de connexion d'électrode (14) de la pluralité de points de connexion d'électrode (14).

14. Implant rétinien selon la revendication 11 ou 12, **caractérisé en ce que** les première et deuxième électrodes sont connectées au même point de connexion d'électrode (14).
